Europäisches Patentamt

European Patent Office

Office européen des brevets

(19)

(11) Publication number: **0 244 211**
**A2**

(12) **EUROPEAN PATENT APPLICATION**

(21) Application number: **87303778.2**

(22) Date of filing: **28.04.87**

(51) Int. Cl.⁴: **C 12 N 5/00**
**// A01N65/00**

(30) Priority: **28.04.86 JP 99165/86**

(43) Date of publication of application:
**04.11.87 Bulletin 87/45**

(84) Designated Contracting States: **CH DE FR GB LI**

(71) Applicant: **NOYAKU BIOTECHNOLOGY KAIHATSU GIJUTSU KENKYU KUMIAI**
**1-8-25 Muromachi, Nihonbashi Chuo-ku**
**Tokyo-to (JP)**

(72) Inventor: **Kawamura, Michio**
**4845-4, Ami Ami-machi**
**Inashiki-gun Ibaraki-ken (JP)**

**Yamazaki, Kiyomi**
**1630-26 Ami Ami-machi**
**Inashiki-gun Ibaraki-ken (JP)**

(74) Representative: **Hildyard, Edward Martin et al**
**Frank B. Dehn & Co. Imperial House 15-19 Kingsway**
**London WC2B 6UZ (GB)**

(54) **Process for mass propagation of plant tissue in vitro.**

(57) A process for mass propagation of Phryma leptostachya Linne which comprises culturing plant tissue of said plant in vitro using a culture medium comprising, as active ingredient, an effective amount of auxin and/or cytokinin. A preferred culture medium for propagating Phryma leptostachya Linne in vitro for the subsequent isolation of the insecticide Haedoxane A obtainable therefrom is disclosed.

**Description**

Process for mass propagation of plant tissue in vitro

The present invention relates broadly to processes for mass propagation of plant tissue in vitro and more particularly to a process for mass propagation of plant tissue of Phryma leptostachya Linne in vitro.

Phryma leptostachya Linne (named as Haedokuso in Japan) is a perennial plant of the family Phrymaceae. This plant has a height of 30-70 cm and grows naturally under the trees in the field and mountain areas of Japan and in other countries such as South East Asia and America. In summer, flowers are found on the flower stalks which grow from the flowering twigs on the top end of the stems. The fruit contains only one seed. After going to seed in late autumn, the aerial portion of the plant dies, while the root remains alive. On the root stalk, two or more dormant buds are formed, which shoot in the following spring.

In Japan, a substance contained in the root of this plant has been used over many years as an insecticide against flies and moreover it has also been known that this substance is capable of killing other insects such as, for example, mosquitoes and moths. A substance having insecticidal activity was isolated from this plant and designated Haedoxane A [Taniguchi et al., Abstracts of the lectures on a joint meeting of the Western Japan Branch of the Japanese Society of Agricultural Chemistry, page 62 (1983)]. It had previously been reported that a substance having insecticidal activity was present in the root, stem, leaf, flower and fruit of this plant, but the active substance itself had not been isolated [Patterson et al., Lloydia, Vol. 38, No. 5, pages 391-403 (Sept-Oct, 1975)].

On the other hand, mass propagation of various plant tissues in vitro by collecting a piece of tissue or mass of the cells from a plant and culturing the same in vitro under sterile conditions is known. Such a process has been applied to, for example, strawberry, lily, begonia, broccoli, cabbage, Bupleurum falcatum (named as Saiko in Japan), camellia and gourd, with reference to, for example, J. Reinert et al., Applied and Fundamental Aspects of Plant Cell, Tissue and Organ Culture, pages 334-356, 1977; J.A. Simmonds et al., Scientia Horticulturae, 5, pages 161-170, 1976; Hartman et al., Plant Propagation, Principle and Practice, 3rd Edit., 1975; U.S. Patent 4,338,745; and Japanese Patent Applications as laid open to public inspection as Kokai 12988/76, 2183/85, 47678/85 and 5727/86.

Methods of culturing plant tissue in vitro, which comprise culturing plant tissue in growth media, with shaking under illumination, is known. For this purpose, various synthetic media such as Murashige-Skoog's medium, Linsmayer-Skoog's medium, White's medium and Knop's medium are widely used, which may, if desired, contain cytokinin (for example, kinetin, zeatin and benzyladenine) and auxin (for example, 2,4-dichlorophenoxyacetic acid, naphthaleneacetic acid, indoleacetic acid and the like). These media may further contain growth-promoting hormones such as gibberellin, abscissic acid and the like. However, no process for mass propagation of Phryma leptostachya Linne plant tissue in vitro has previously been described. The present invention is based upon the discovery that mass propagation of the plant tissue of Phryma leptostachya Linne in vitro is possible by using a medium comprising an effective amount of auxin and/or cytokinin.

According to the present invention, there is provided a process for mass propagation of Phryma leptostachya Linne which comprises culturing plant tissue of said plant in vitro using a culture medium comprising an effective amount of auxin and/or cytokinin.

Preferably the culture medium comprises 0.001-50 ppm of auxin and/or 0.005-150 ppm of cytokinin.

The media which may be used for the purpose of the present invention contain, like in the cases of known media used for culturing plant tissues, various substances required for plant growth such as, for example, carbohydrate (e.g. sucrose), organic acid (e.g. fatty acid), ethanol and other carbon source; inorganic or organic source of nitrogen; and inorganic salts. The media may further contain trace amounts of metallic ions, amino acids, vitamins and the like. Usually, the media contain approximately 5-100 g/l of carbon source, 0.1-10 g/l of nitrogen source, 0.001-1000 mg/l of inorganic substances, and 0.01-30 mg/l each of vitamins and amino acids.

The term "auxin" denotes a substance capable of inducing the elongation and growth of plant stems and leaves and has the same physiological activity as indole-3-acetic acid. Auxin is capable of increasing the permeability of cell membranes, promoting cell division, inhibiting the growth of lateral buds, and promoting the formation of adventitious roots and flower buds. In general, auxin exerts its promoting activities at lower concentration and its inhibiting activities at higher concentrations. Its activities may further vary, depending on factors such as, for example, the types, organs and physiological ages of the plants.

The term "cytokinin" denotes substances having the same physiological activity as kinetin, such as derivatives thereof containing 6-amino purine in the fundamental structure, of which the amino group is modified with a substituent which usually carries 5 carbon atoms. A typical naturally-occurring cytokinin is exemplified by zeatin. Cytokinin is capable of promoting the elongation of buds and the growth of leaves, and of inhibiting senility, accumulation of amino acids and opening of the stoma. It is well known that both auxin and cytokinin are intimately concerned with the differentiation of plants.

Culturing may usually be carried out at a temperature of from 20 to 30°C.

In the following specification, some embodiments of the process of the present invention are described by the use of Murashige-Skoog's medium (hereinafter referred to as MS medium) which contains as auxin l-naphthaleneacetic acid or indole-3-acetic acid (hereinafter referred to as NAA and IAA respectively) and/or as cytokinin benzyladenine (hereinafter referred to as BA), although it is possible, if desired, to use other media without difficulty with reference to the following specification.

(A) Preparation of materials for culturing plant tissue in vitro e.g. a piece of plant tissue or a mass of plant cells:

(I) A seed is cultured by using a suitable medium in conventional manner to obtain a bud, of which the top portion is then collected under sterile conditions and used for subsequent culturing.

(2) A dormant bud is collected from the body of the root and is aroused from dormancy in conventional manner. The top portion of the bud is collected under sterile conditions and is then cultured in conventional manner until the growth of the top portion is observed with unaided eyes. The resultant material is used for culturing.

(3) It is also possible, if desired, to collect the stem, leaf or root and cut into pieces. The piece is cultured in vitro by using a suitable medium to obtain adventitious buds. The piece having the adventitious buds or the adventitious buds themselves collected therefrom may be used for culturing.

Where a medium specially designated as one for culturing plant tissue in vitro is used in the above-mentioned procedure, it is possible to carry out the culturing after addition of auxin and/or cytokinin to the medium used.

(B) Culturing:

As a result of various experiments carried out by us, it has been revealed as follows:-

(I) For example, where MS medium (concentration I/2; containing 3% sucrose; pH 6.2), to which 0.0I-I5 ppm of auxin and/or 0.I-I50 ppm of cytokinin, is used for culturing a sample at a temperature of 25°C under illumination of 2500 lux, about 3 weeks after the beginning of culturing, a I0 fold increase of the number of axillary buds is found.

(2) By culturing in a similar manner to that described above except that the amount of cytokinin used is 0.005-I5 ppm, 3-5 weeks after the beginning of culturing, the elongation and growth of the aerial portion is observed.

(3) By culturing in a similar manner to that described above except that 0.I-2.5 ppm of auxin and 0.005-5 ppm of cytokinin is used, the root grows continuously to result in an increase of root weight to more than about I0% by weight of the whole plantlet.

For example, where grown axillary buds (0.5 ± 0.2 g) were cultured in a liquid medium (MS medium; I00 ml) for 30-40 days at a temperature of 25°C in a similar manner to that described above, the root portion amounted to 30-40% of the wet weight (about 2-4 g) of the resultant plantlet as a whole.

Examples of preferred concentrations of auxin and cytokinin used for culturing the tissues of root, leaf and stem are shown in the following table.

## TABLE 1

| Tissue used | Desired product | Auxin (ppm) | Cytokinin (ppm) |
|---|---|---|---|
| Root | Elongated root | 0.005-5 | 0.005-5 |
| Root | Grown stem and leaf | 0.001-0.5 | 0.05-50 |
| Stem | Elongated root | 0.005-5 | 0.005-5 |
| Stem | Grown stem and leaf | 0.005-5 | 0.05-50 |
| Leaf | Elongated root | 0.005-5 | 0.005-0.5 |
| Leaf | Grown stem and leaf | 0.005-5 | 0.05-50 |

In the case where each reagent is used alone, it is preferred to use, for example, 0.00I-50 ppm of auxin or 0.005-I50 ppm of cytokinin.

Better results may be obtained by adding at least one growth-promoting hormone, such as gibberellin (for example, not more than 3 ppm) to the above-mentioned medium. It is also possible, if desired, to use agar medium instead of liquid medium.

No observable decrease of the amount of active substance was noted, for example, where subculturing was continued over 20 generations.

3

It is preferred to use, for example, the following growth conditions with MS media:-

TABLE 2

| Tissue | Period (days) | Conditions for culturing |
|---|---|---|
| Growth of axillary buds | 20-30 (25°C) | concentration 1/2; containing sucrose (3%), NAA (1 ppm) and BA (10 ppm); pH 6.0 (If desired 0.8% agar may be added) |
| Rooting | 30-40 (25°C) | concentration 1/10; containing no $NH_4NO_3$, no EDTA, sucrose (1%) and NAA (0.1 ppm); pH 6.0 (If desired not more than 0.1 ppm of BA may be used) |

It has been found that the desired insecticide is mainly contained in the root. For the purpose of obtaining a larger amount of the root, the following preferred medium (PM) is particularly suitable (mg/l):-

$KNO_3$ 190; $CaCl_2.2H_2O$ 4.4; $MgSO_4.7H_2O$ 37; $KH_2PO_4$ 1.7; $FeSO_4.7H_2O$ 2.78; $H_3BO_3$ 0.62; $MnSO_4.4H_2O$ 0.223; $ZnSO_4.7H_2O$ 0.086; KI 0.0083; $Na_2MoO_4.2H_2O$ 0.0025; $CuSO_4.5H_2O$ 0.0025; $CoCl_2.6H_2O$ 0.00025; thiamine-HCl 0.004; myo-inositol 1; pyridoxine-HCl 0.05; nicotinic acid 0.005; glycine 0.002; in aqueous solution pH 6.0-6.5.

The medium may comprise 0.01-50 ppm of auxin and/or not more than 50 ppm (preferably not more than 5 ppm) of cytokinin.

The concentrations of the ingredients may vary within a range of from 1/20 to ×20, depending upon various factors such as, for example, culturing conditions and physiology of the tissue used [Thus in Example 4, this medium is used at a higher concentration (×3)].

It is particularly preferred to use a growth medium containing auxin and which is substantially free of both ammonium nitrate and EDTA. More preferably such a medium contains 0.01-50 ppm auxin. Such media are of advantage for culturing P. leptostachya tissue in vitro, particularly root tissue, and the cultured tissue is found to contain the desired active substance.

The substance having insecticidal activity is present, for example, in the stem, leaf and root. The following table shows the results obtained by isolating the insecticidal substance from the root of naturally-occurring plant as well as from the root and aerial portion (stem and leaf) of a plant cultured in vitro. The determination was effected with reference to the method of Taniguchi et al. [Agric. Biol. Chem., 36, 1013 (1972)].

TABLE 3

| Origin | Product | $LD_{50}$ ($\mu g/g$) |
|---|---|---|
| Root (naturally-occurring | Crude | 0.014 |
| | Purified | 0.006 |
| Root (tissue-cultured) | Crude | 0.016-0.010 |
| Stem and leaf (tissue-cultured) | Crude | 0.021 |

Note: Topical application to houseflies (female) with reference to Fukami et al., Nogyo Jikken-ho, page 73, published by Soft Science K.K., Japan (1981) in Japanese version

Where the method of Taniguchi et al. was applied to isolate the substance having insecticidal activity from the roots of naturally-occurring plant and tissue cultured plant of Phryma leptostachya Linne, a large amount of inactive material was isolated, more than 10 times as much as the corresponding amount from the tissue-cultured plant. Also, the amount of the active substance isolated from the tissue-cultured plant was at least equal to the corresponding amount isolated from the naturally-occurring plant.

By the process of the present invention, it is possible to obtain a large amount of Phryma leptostachya Linne having improved properties in a shorter period of time. The plant thus-obtained contains at least as much insecticide as a natural plant, and moreover contains a very small amount of undesired impurities.

Example I

I00 seeds of Phryma leptostachya Linne were treated with gibberellin (I000 ppm) for I8 hours to arouse from dormancy. The seeds were then sterilized by treating with ethanol (70%) for 3 minutes, followed by treating with sodium hypochlorite (0.5%) for 30 minutes. Germination of the sterilized seeds was effected by allowing them to stand in an incubator at a temperature of 25°C for I0 days. There were obtained 85 sterilized plantlets. I0 sterilized buds thus-obtained were cultured at a temperature of 25°C for 3 weeks under illumination of 2500 lux by the use of an MS medium (I0 ml; concentration I/2; containing 3% sucrose and 0.8% agar; with addition of 0.I ppm of NAA as auxin and I0 ppm of BA as cytokinin; pH 6.2) to obtain I32 axillary buds. A suitable number of the resultant axillary buds were collected and subcultured for a period of one month. As a result from continuous subculturing over I0 generations, 25 axillary buds were obtained. The axillary buds were cultured in a similar manner to that described above with the exception that NAA (0.I ppm) and BA (I ppm) were added to the medium (I00 ml) containing no agar. 4 weeks after the beginning of culturing, the elongation and growth of the aerial portion were observed. However, a small amount of the root was found.

The aerial portion (stem and leaf; 0.45 g) thus obtained was cultured for 3 weeks in a similar manner to that described above except the NAA in the medium (I00 ml) was I ppm. Elongation and growth of the roots were observed. The weight of the resultant roots was 2I.3% of the weight of the plantlet as a whole (5.46 g).

It was possible to prepare a large amount of axillary buds by continuing subculturing many times. No observable relationship was noted between the subculturing time and the amount of the insecticidal substance contained in the root.

Example 2

Plantlets obtained by germination of sterilized seeds were used to prepare pieces of the stem (length 5-6 mm). They were cultured at a temperature of 25°C under illumination of 2500 lux by the use of an MS medium [I0 ml; concentration I/2; containing 3% sucrose and 0.8% agar; with addition of NAA (0.I ppm) as auxin and BA (0.I ppm) as cytokinin; pH 6.2]. About 2 weeks after the beginning of culturing, rooting was found, and about I.5 months later, 5 roots were formed, of which average length was 4.5 cm.

Example 3

Separately, pieces of stem (length 5-6 mm) were cultured in a similar manner to that described above with the exception that the medium (l0 ml) containing NAA (0.l ppm) and BA (l0 ppm). 3 weeks later, germination of adventitious buds was observed, and l.5 months later, 6 adventitious buds were obtained.

Example 4

In a similar manner to that described in Example l, an aerial portion (0.56 g) was cultured by using a PM medium [l00 ml; concentration ×3; containing BA (0.3 ppm), NAA (l ppm) and sucrose (l%); pH 6.0]. About 5 weeks after the beginning of culturing, elongation and growth of the root portion were noted. 32% of the resultant plantlet (2.43 g) was occupied by the root.

Experiments

From the aerial portion (stem and leaf) (0.5l g) and root (0.46 g) of plantlets obtained by the methods of Examples l and 2 respectively, insecticidal substance was isolated with reference to the method of Taniguchi et al. On each occasion, the active material was collected and dissolved in 0.5 ml of isopropylether. The sample was subjected to high performance liquid chromatography using a solvent system of isopropylether/ether acetate (9:l v/v; silica gel) to isolate the active fractions, which were combined, concentrated and dried to obtain the desired product in the form of powders (l6 ug from the aerial portion and 44 ug from the root).

In one experiment 626 $\mu$g/g and 50.9 $\mu$g/g (on dry basis) of the active substance (crude) were respectively obtained from the root and aerial portion of a tissue-cultured plant. Moreover, it was noted that the plants obtained by the process of the present invention contain a larger amount of the active substance (for example, about ×l.5 by weight) in comparison with the corresponding amount obtained from naturally-occurring plants.

**Claims**

1. A process for mass propagation of Phryma leptostachya Linne which comprises culturing plant tissue of said plant in vitro using a culture medium comprising, as active ingredient, an effective amount of auxin and/or cytokinin.

2. A process according to claim l, wherein the medium comprises 0.00l-50 ppm of auxin.

3. A process according to claim l or claim 2, wherein the medium comprises 0.005-l50 ppm of cytokinin.

4. A process according to any one of the preceding claims wherein the medium further comprises an effective amount of at least one growth-promoting hormone.

5. A process according to claim 4 wherein the medium comprises not more than 3 ppm gibberellin.

6. A process according to any one of the preceding claims wherein the plant tissue is root tissue and the medium comprises 0.0l-50 ppm auxin and is substantially free of both $NH_4NO_3$ and EDTA.

7. A process according to claim 6 wherein the medium comprises not more than 50 ppm cytokinin.

8. A process as claimed in claim 7 wherein the medium comprises not more than 5 ppm cytokinin.

9. Use of a culture medium as defined in any one of claims l to 8 for mass propagation of Phryma leptostachya Linne plant tissue in vitro.